(19) Europäisches Patentamt
European Patent Office
Office européen des brevets

(11) Veröffentlichungsnummer: **0 484 784 A1**

(12) **EUROPÄISCHE PATENTANMELDUNG**

(21) Anmeldenummer: **91118344.0**

(22) Anmeldetag: **28.10.91**

(51) Int. Cl.5: **C09B 5/62**, C09D 17/00, C08K 5/00

(30) Priorität: **03.11.90 DE 4035009**

(43) Veröffentlichungstag der Anmeldung:
**13.05.92 Patentblatt 92/20**

(84) Benannte Vertragsstaaten:
**CH DE FR GB LI**

(71) Anmelder: **HOECHST AKTIENGESELLSCHAFT**
**Postfach 80 03 20**
**W-6230 Frankfurt am Main 80(DE)**

(72) Erfinder: **Tröster, Helmut, Dr.**
**Am Erdbeerstein 44**
**W-6240 Königstein/Taunus(DE)**

(54) **Schwarze Perylen-3,4,9,10-tetracarbonsäurediimide, ihre Herstellung und Verwendung.**

(57) Die Erfindung betrifft Perylen-3,4,9,10-tetracarbonsäurediimide der Formel (I)

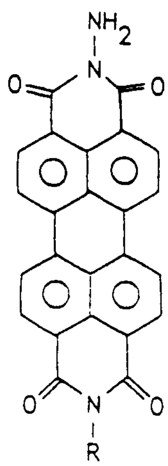

(I)

in der R den 3- und/oder 4-Methoxybenzylrest bedeutet, sowie ihre Herstellung und Verwendung als schwarze Pigmente, unter anderem in Lackzubereitungen, Druckfarben oder zur Färbung von Polymeren.

EP 0 484 784 A1

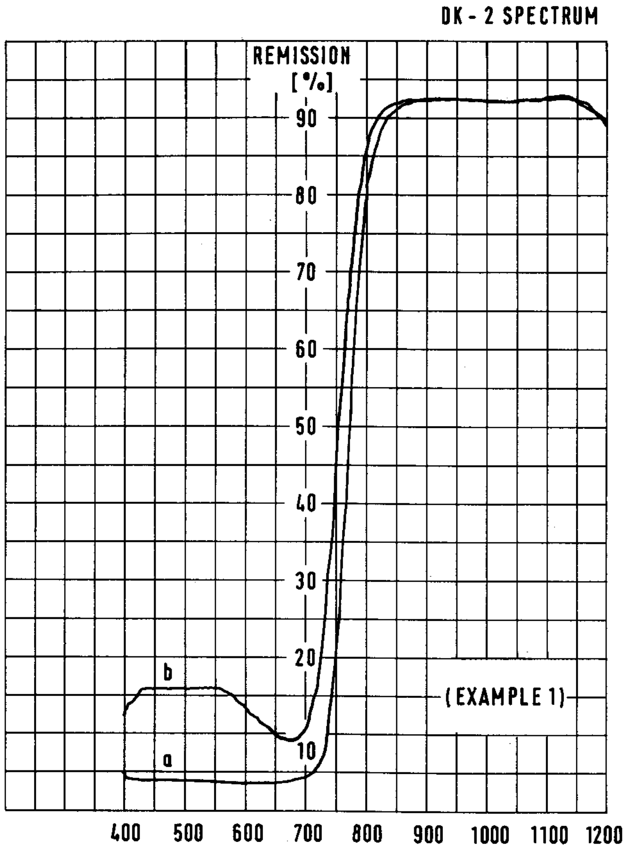
DK - 2 SPECTRUM
REMISSION
[%]
90
80
70
60
50
40
30
20
10
b
a
(EXAMPLE 1)
400
500
600
700
800
900
1000
1100
1200
λ[nm]

FIG. 1

Aus der großen Zahl von Perylentetracarbonsäurediimid-Farbmitteln sind nur wenige Verbindungen bekanntgeworden, die eine schwarze Farbe aufweisen und als schwarze Pigmente Verwendung finden können. Sie sind in der DE-Offenlegungsschrift 2451780, GB-A-1523415, GB-A-1537358 DE-A 3422757 und GB-A-2177103 sowie in der US-PS-4450273 beschrieben. Hierbei handelt es sich ausschließlich um symmetrisch N,N'-substituierte Perylentetracarbonsäurediimide. Die geringe Zahl solcher spezieller Pigmente erklärt sich durch besondere strukturelle Voraussetzungen, die vom Farbstoffmolekül erfüllt sein müssen, um die den Farbeindruck schwarz bedingende Anordnung der Moleküle im Kristallgitter zu ermöglichen.

Aus der Literatur [Y. Nagao et al., Dyes and Pigments 5 (1984), 179-181, Tab. 3; desgl. 6 (1985), 309, Tab. 3] sind in jüngster Zeit auch zahlreiche asymmetrisch N,N'-substituierte Perylentetracarbonsäurediimide bekannt geworden. Unter ihnen befindet sich jedoch kein schwarzes Pigment.

Die Erfindung betrifft nun Perylen-3,4,9,10-tetracarbonsäurediimide der Formel (I) gemäß Anspruch 1, sowie ihre Herstellung und ihre Verwendung als schwarze Pigmente.

Wie in der zitierten GB-A-2177103 ausgeführt, kann aufgrund einer gegebenen Molekülstruktur keine Voraussage auf die zu erwartende Pigmentfarbe gemacht werden. Es war daher im Falle der erfindungsgemäßen Diimide nicht vorhersehbar, daß sich auch bei einem asymmetrischen Molekülaufbau schwarze Pigmente finden lassen würden.

Die neuen, asymmetrischen Perylentetracarbonsäurediimide (I) sind sehr gut als schwarze Pigmente für Lacke auf Lösungsmittel- oder Wasserbasis und Druckfarben sowie auch für Polymere, wie z.B. Polyvinylchlorid und Polyethylen, geeignet.
Die damit erhaltenen tiefschwarzen bzw. im Verschnitt mit beispielsweise Weißpigmenten grauen Farbtöne zeichnen sich durch sehr gute Allgemeinechtheiten, insbesondere ausgezeichnete Licht- und Wetterechtheit aus. Aufgrund der Remissionswerte im Rot-und nahen Infrarotspektralbereich, die denen des Chlorophylls sehr ähnlich sind, können die Pigmente auch besonders gut bei der Herstellung von Tarnfarben eingesetzt werden. Gegenüber den Schwarz-Pigmenten der US-PS-4450273 zeichnen sie sich vor allem durch eine höhere Farbstärke aus.

Zur Herstellung der Verbindungen der Formel (I) werden Perylen-3,4,9,10-tetracarbonsäuremonoimide der Formel (II)

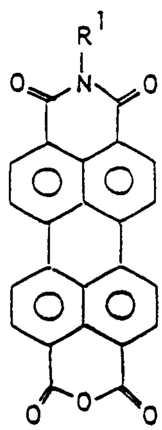

(II)

in an sich bekannter Weise mit Aminen der Formel (III)

$R^2$ - $NH_2$ (III)

kondensiert.

$R^1$ und $R^2$ haben dabei in diesen Formeln die folgende Bedeutung: Steht $R^1$ in Formel (II) für die Aminogruppe, so bedeutet $R^2$ in Formel (III) den 3- und/oder 4-Methoxybenzylrest und umgekehrt.

Die Umsetzung kann man in Wasser und/oder organischen Lösungsmitteln, wie Methylglykol, Glykol oder Chinolin bei erhöhter Temperatur, im allgemeinen bei Temperaturen von 100 bis 250 °C, vorzugsweise von 120 bis 200 °C, unter Normaldruck oder gegebenenfalls auch unter erhöhtem Druck, vornehmen.

Die Verbindung (III) wird zumindest in äquimolaren Mengen, bevorzugt jedoch im Überschuß von 20 bis 100 mol-% eingesetzt. Das in üblicher Weise isolierte Reaktionsprodukt kann gegebenenfalls durch geeignete, übliche Finishmethoden, wie Mahlung beispielsweise gemäß der US-Patentschrift 3615800, oder Umfällung aus Schwefelsäure und gegebenenfalls nachfolgende Lösungsmittelbehandlung in die gewünschte Pigmentform überführt werden.

Die als Ausgangsverbindungen benötigten Monoimide der Formel (II) können beispielsweise nach dem in der US-PS-4599408 beschriebenen Verfahren, auf die hiermit Bezug genommen wird, hergestellt werden, in dem man die entsprechenden Perylen-3,4,9,10-tetracarbonsäuremonoanhydridmonoalkalisalze mit Verbindungen $R^1$-$NH_2$ umsetzt, in der $R^1$ entweder die Bedeutung von R hat oder für die Aminogruppe steht.

Die Erfindung wird anhand der nachfolgenden Beispiele näher erläutert.

I. Herstellungsbeispiele

1.) Herstellung der Verbindung gemäß der Formel

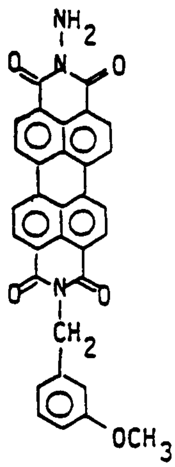

In eine Lösung von 200 ml Methylglykol und 100 ml Wasser wurden 8,5 g Dihydraziniumsulfat (95%ig) eingetragen und anschließend 10,8 g 50%ige Kalilauge zugegossen. Zu dieser Mischung wurden 15,3 g Perylen-3,4,9,10-tetra-carbonsäuremono-3'-methoxybenzylimid gegeben und im Autoklav 3 h bei 130-140 °C umgesetzt.

Das Reaktionsprodukt wurde bei Raumtemperatur abgesaugt, mit Methylglykol/Wasser 1:1 bis zum farblosen Filtratablauf und schließlich mit Wasser gewaschen und getrocknet. Es wurden 15,6 g (99,0 % d. Th.) des Zielproduktes in Form eines schwarzen Kristallisates mit grünlichem Oberflächenglanz erhalten.

| | | | | |
|---|---|---|---|---|
| Analyse: | ber. | C 73,1 % | H 3,6 % | N 8,0 % |
| $C_{32}H_{19}N_3O_5$ | gef. | C 72,9 % | H 3,6 % | N 7,8 % |
| Mol 525,5 | | | | |

m/e = 525 ($M^+$) (massenspektrom. bestimmte Molmasse)

Zur Überführung in die Pigmentform wurden 30 g des erhaltenen Rohpigmentes in einem Mahlbehälter mit ca. 1 l Inhalt, beschickt mit 300 ml Aceton und 1200 g Quarzitperlen, ø 2-3 mm, auf einer Schwingmühle 12 Stunden gemahlen. Das Mahlgut wurde über ein Sieb von den Perlen getrennt und die erhaltene Pigmentsuspension im Rotationsverdampfer zur Trockne gebracht. Es wurde in praktisch quantitativer Ausbeute ein tief schwarzes Pigmentpulver erhalten.

2.) Herstellung der Verbindung gemäß der Formel

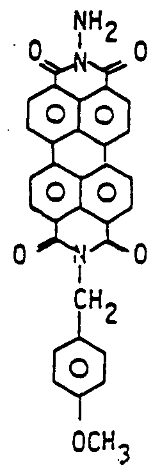

Es wurde wie in Beispiel 1 verfahren, jedoch wurde anstelle von Perylen-3,4,9,10-tetracarbonsäure-mono-3'-methoxybenzylimid die gleiche Menge des entsprechenden 4'-Methoxybenzylimids verwendet. Es wurden 15,3 g (97,7 % d. Th.) des entsprechenden schwarzen Diimids erhalten.

| | | | | |
|---|---|---|---|---|
| Analyse: | ber. | C 73,1 % | H 3,6 % | N 8,0 % |
| $C_{32}H_{19}N_3O_5$ | gef. | C 73,5 % | H 3,7 % | N 8,1 % |
| Mol 525,5 | | | | |

m/e = 525 ($M^+$)
Die Mahlung des Rohpigmentes erfolgte wie in Beispiel 1 angegeben.
3.) 20,3 g Perylen-3,4,9,10-tetracarbonsäure-mono-N-aminoimid wurden in 300 ml Chinolin in Gegenwart von 5,0 g Zinkacetat mit 27,4 g 3-Methoxybenzylamin in 6 h bei 200-210 °C unter Abdestillieren des Reaktionswassers umgesetzt. Das schwarze Reaktionsprodukt wurde abgesaugt, mit Chinolin, Methanol und Wasser gewaschen und getrocknet.
Ausbeute: 24,5 g (93,3 % d. Th.).
Das erhaltene schwarze Rohpigment entsprach dem nach Beispiel 1 erhaltenen Produkt.
4) Herstellung der Verbindungen gemäß der Formel

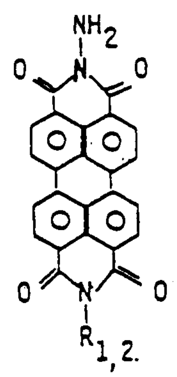

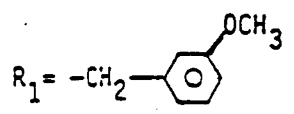

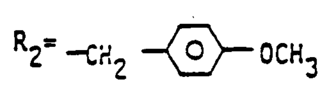

$R_1:R_2$ ca. 4:1

Eine Mischung von 12,3 g Perylentetracarbonsäuremono-3'-methoxybenzylimid und 3,1 g Perylentetracarbonsäuremono-4-methoxybenzylimid wurde in eine Lösung von 9,8 g Hydraziniumsulfat (99 %ig) und 16,0 g 50%iger Kalilauge in 400 ml 60%igem Ethanol eingetragen und anschließend im Autoklav 5 h bei 130-140 °C zur Reaktion gebracht. Das schwarze Kristallisat wurde abgesaugt, mit Wasser gewaschen und getrocknet. Die Ausbeute betrug 15,8 g (99,8 % d. Th.).
m/e : 525 ($M^+$)

II) Anwendungsbeispiele

a) Herstellung einer 5%igen Vollton-Einbrennlackierung:

Eine Mischung von 62,5 ml Quarzitperlen, ø 3 mm, 4,5 g des Pigmentes gemäß Beispiel I.1 bzw. I.2 und 25,5 g eines handelsüblichen Alkydharzlackes (35 Gew.-% Feststoffgehalt) wurden in einem Kunststoffbecher 30 Minuten auf dem "Paintshaker" dispergiert. Anschließend wurden 60 g eines handelsüblichen Alkyd-Melaminharzlackes (55,8 Gew.-% Feststoffgehalt) zugegeben und nach einer weiteren fünfminütigen Dispergierung die Quarzitperlen abgesiebt. Die Lacke wurden mit einem "Handcoater" (Nr. 8) in einer Schichtdicke von 100 µm auf weißen Karton aufgetragen und 30 Minuten bei 140 °C eingebrannt.

b) Herstellung einer Einbrennlackierung in der Weiß-Aufhellung 1:5

12,0 g der gemäß a) hergestellten beiden Volltonlacke wurden mit je 10 g eines handelsüblichen weißen Alkyd-Melaminharzlackes mit einem Gehalt von 30 Gew.-% Titandioxid vermischt. Die so erhaltenen Lacke wurden, wie in a) angegeben, mit einem "Handcoater" auf weißen Karton aufgetragen und eingebrannt.

Von den nach a) und b) erhaltenen Lackierungen wurden die Remissionskurven aufgezeichnet. Der Kurvenverlauf ist in den Abbildungen 1 (Beispiel 1) und 2 (Beispiel 2) im Anhang wiedergegeben. Kurve a entspricht jeweils der Volltonlackierung, Kurve b der Weißaufhellung.
Die Messung der Reflektionsspektren im Wellenlängenbereich von 400 bis 1200 nm erfolgte mit einer Bleisulfidzelle an einem Spektralphotometer DK 2 der Firma Beckmann Instruments gegen Bariumsulfat als Weißstandard.

Gegenüber dem gemäß Beispiel 1 der US-PS-4450273 und Schwingmahlung gemäß obigem Beispiel I.1 erhaltenen Pigment sind die Lackierungen des jeweiligen Weißverschnitts der erfindungsgemäßen Pigmente farbstärker.

**Patentansprüche**

1. Perylen-3,4,9,10-tetracarbonsäurediimide der Formel (I)

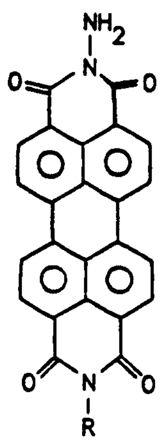

(I)

in der R den 3- und/oder 4-Methoxybenzylrest bedeutet.

2. Verfahren zur Herstellung der Perylen-3,4,9,10-tetracarbonsäurediimide (I) gemäß Anspruch 1, dadurch gekennzeichnet, daß man Perylen-3,4,9,10-tetracarbonsäuremonoimide der Formel (II)

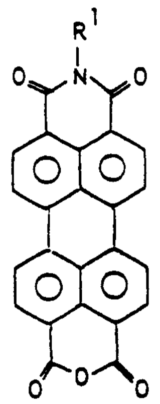

(II)

mit Aminen der Formel (III),

$R^2$ - $NH_2$ (III)

umsetzt, wobei $R^1$ in Formel (II) entweder die gleiche Bedeutung wie R gemäß obiger Formel (I) hat und dann $R^2$ in Formel (III) für die Aminogruppe steht oder $R^1$ die Aminogruppe bedeutet und dann $R^2$ die gleiche Bedeutung wie R besitzt.

**3.** Verwendung der Perylen-3,4,9,10-tetracarbonsäurediimide (I) gemäß Anspruch 1 als schwarze Pigmente.

**4.** Lackzubereitungen, Druckfarben oder Polymere, welche die Perylen-3,4,9,10-tetracarbonsäurediimide (I) gemäß Anspruch 1 enthalten.

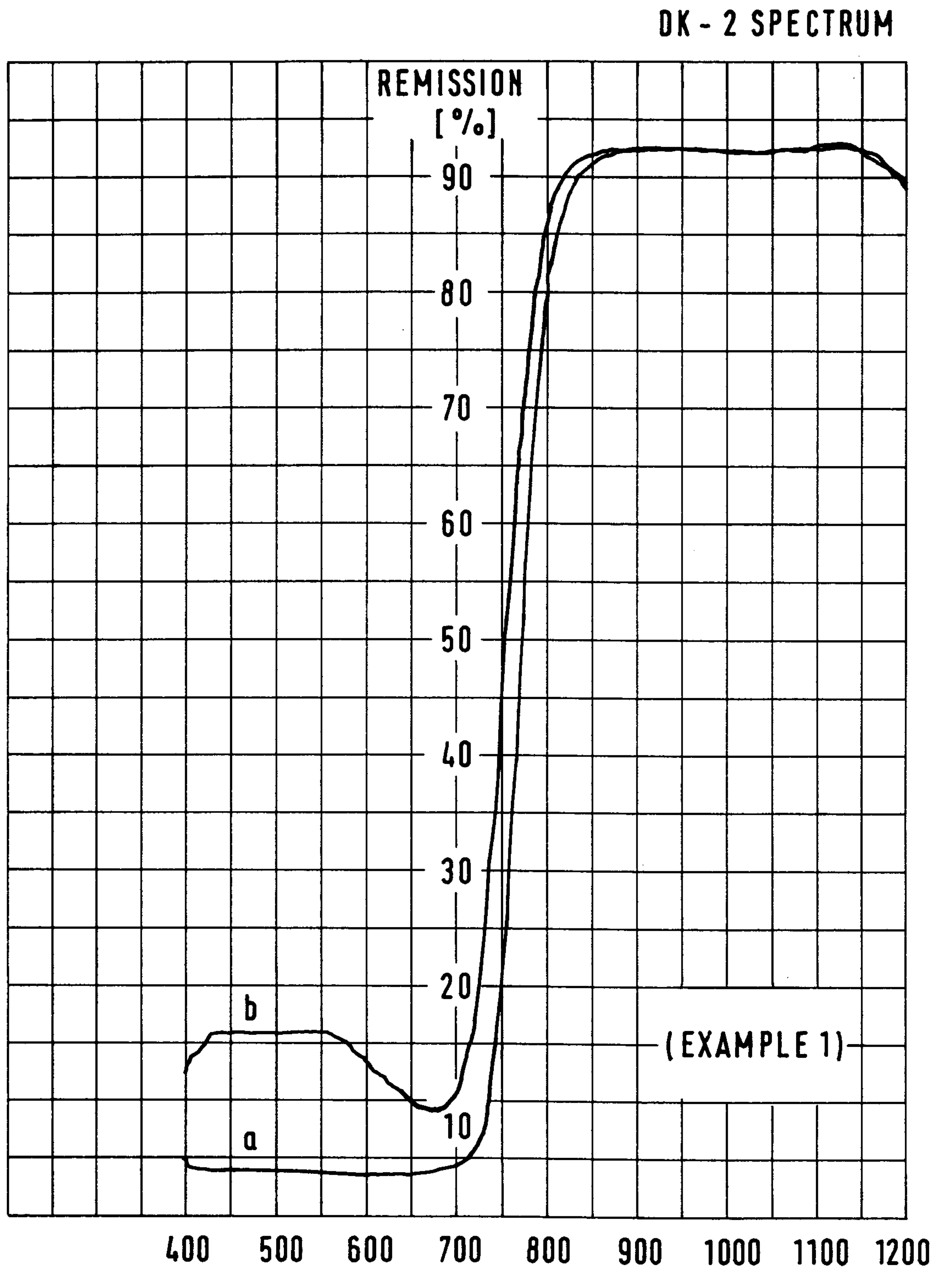
DK - 2 SPECTRUM
REMISSION
[%]
90
80
70
60
50
40
30
20
10
b
a
(EXAMPLE 1)
400
500
600
700
800
900
1000
1100
1200
λ[nm]

FIG. 1

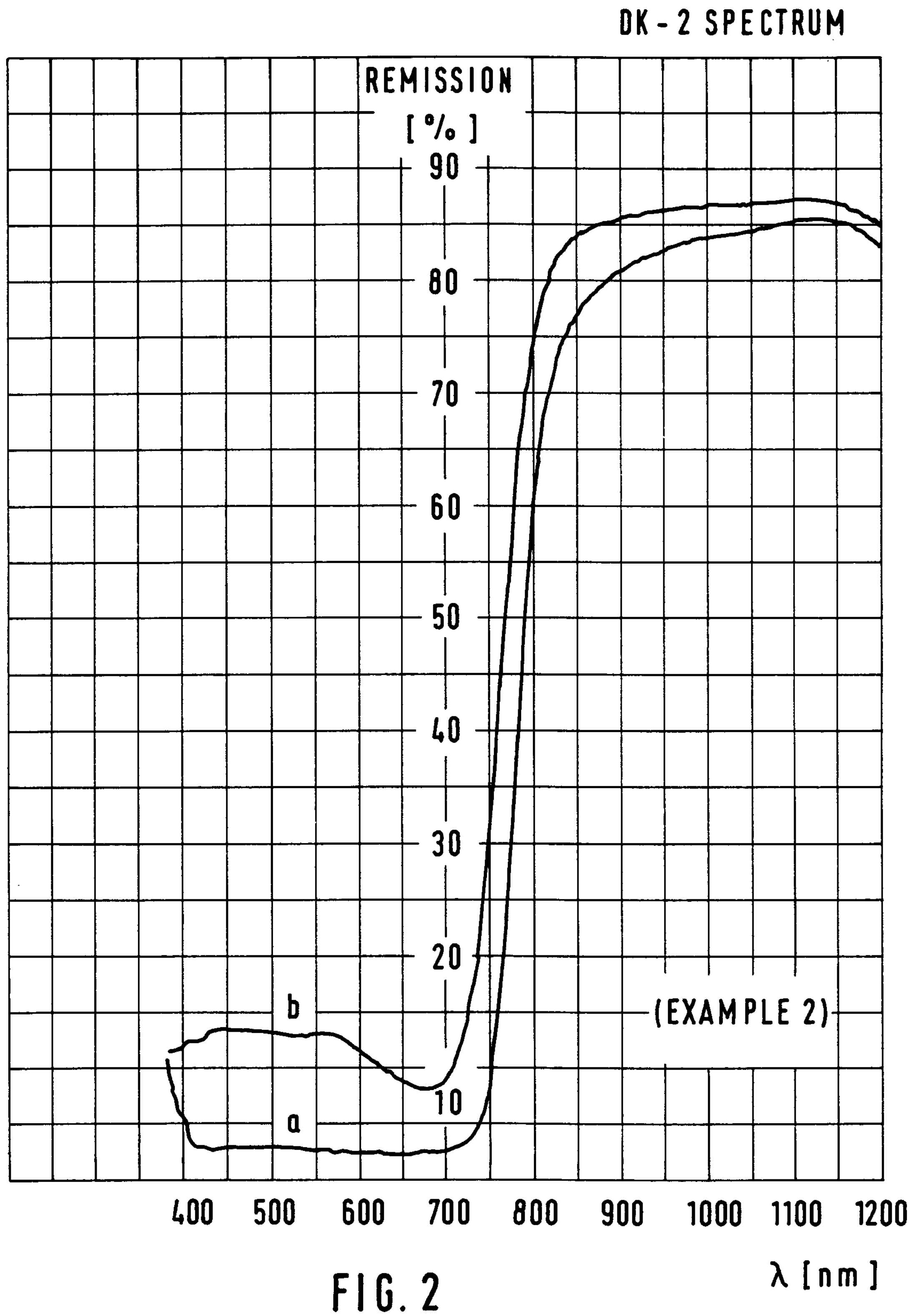
DK - 2 SPECTRUM
REMISSION
[%]
90
80
70
60
50
40
30
20
10
b
a
(EXAMPLE 2)
400
500
600
700
800
900
1000
1100
1200
λ [nm]

FIG. 2

Europäisches Patentamt

# EUROPÄISCHER RECHERCHENBERICHT

Nummer der Anmeldung

EP 91118344.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,A | US - A - 4 450 273 (GRASER) * Zusammenfassung; Textspalte 2, Zeilen 28-32 * -- | 1-4 | C 09 B 5/62<br>C 09 D 17/00<br>C 08 K 5/00 |
| D,A | GB - A - 1 537 358 (BASF AKTIENGESELLSCHAFT) * Patentansprüche; Seite 2, Zeilen 26-28 * -- | 1-4 | |
| D,A | DE - B - 2 451 780 (BASF AG) * Patentanspruch; Spalte 1, Zeilen 51-56 * ---- | 1-4 | |

RECHERCHIERTE SACHGEBIETE (Int Cl 5)

C 09 B
C 09 D
C 08 K

Der vorliegende Recherchenbericht wurde für alle Patentansprüche erstellt.

| Recherchenort | Abschlußdatum der Recherche | Prüfer |
|---|---|---|
| WIEN | 03-02-1992 | HAUSWIRTH |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 62